# EUROPÄISCHE PATENTSCHRIFT

(11) **EP 1 076 540 B1**
(45) Veröffentlichungstag und Bekanntmachung des Hinweises auf die Patenterteilung: **14.07.2004**
(21) Anmeldenummer: 99917993.0
(22) Anmeldetag: 19.04.1999
(51) Int. Cl.: A61F 13/08

(54) **KOMPRESSIONSVERBAND**
COMPRESSION BANDAGE
BANDAGE COMPRESSIF

(30) Priorität: 07.05.1998 DE 29808232 U
(43) Veröffentlichungstag der Anmeldung: 21.02.2001
(73) Patentinhaber: Lohmann GmbH & Co. KG, 56567 Neuwied (DE)
(72) Erfinder: FELDGIEBEL, Rainer, D-56566 Neuwied (DE)
(74) Vertreter: Flaccus, Rolf-Dieter, Dr.
(86) Internationale Anmeldenummer: PCT/EP1999/002608
(87) Internationale Veröffentlichungsnummer: WO 1999/056683

(56) Entgegenhaltungen:
- EP-A- 0 286 554
- GB-A- 295 886
- GB-A- 680 670
- US-A- 2 687 723
- US-A- 3 605 122
- US-A- 4 367 733
- US-A- 5 139 479
- US-A- 5 620 413
- US-A- 5 676 641

## Beschreibung

Die Erfindung betrifft einen Kompressionsverband zum Stützen des Gewebes im Bereich zwischen Fuß und Knie, beispielsweise zur Behandlung venöser und/oder lymphatischer Schwellungen.

Bei diagnostischer Indikation eines venös bedingten Ödems oder eines Lymphödems ist die Extremität aufgrund von Flüssigkeitsansammlungen geschwollen. Diese Schwellungen müssen unter anderem durch Anlegen eines Kompressionsverbandes eingedämmt und nach und nach beseitigt werden, wobei der Umfang der Wade durch Abschwellen geringer wird. Diese Erscheinung wird medizinisch als Akutphase bezeichnet. In dieser Akutphase wird überwiegend eine Behandlung mit Kompressionsbinden empfohlen. Wegen der beim Anlegen von Kurzzug-Kompressionsbinden zu überwindenden Schwierigkeiten - dabei ist beispielsweise eine exakt definierbare Vorspannung des Stretch-Kompressionsverbandes einzuhalten - muß das Anlegen des Verbandes zumeist von Fachpersonal oder vom Arzt durchgeführt werden, weil das Anwickeln für den Patienten als zu schwierig angesehen wird. Vor allem das Wickeln des Vorfußes und der Ferse gelten als besonders schwierig.

Nach Abschwellen der Extremität aus der Akutphase wird in der dann folgenden Erhaltungsphase in den meisten Fällen ein Kompressionsstrumpf verordnet. Dieser muß in ca. 40 % der Fälle maßgefertigt werden, weshalb der Strumpf außerordentlich teuer wird.
Bei den vorgenannten venösen und lymphatischen Erkrankungen handelt es sich zumeist um chronische Krankheiten, die fallweise lebenslang behandelt werden müssen. In dieser Zeit wechseln sich bei den meisten Patienten Akut- und Erhaltungsphasen ab, weil insbesondere in Sommermonaten oder bei schlechter Disziplin der Patienten die betroffenen Extremitäten zeitweilig stark anschwellen. Dies führt zu dem gravierenden Nachteil, daß der verordnete Kompressionsstrumpf dann nicht mehr paßt und durch eine vom Fachpersonal anzulegende Kompressionsbinde mindestens zeitweilig ersetzt werden muß.

GB 295 886 offenbart einen Kompressionsverband zum Stützen des Gewebes im Bereich zwischen Fuß und Knie, beispielsweise zur Behandlung venöser und/oder lymphatischer Schwellungen, in Sockenform mit einem fertigen Fußteil, welches ein rundgestricktes Kompressionsmittelteil aufweist und oberhalb des Knöchels abschließt.

Der Erfindung liegt die Aufgabe zugrunde, einen verbesserten Kompressionsverband anzugeben, bei welcher unter Überwindung der vorgenannten Nachteile und Schwierigkeiten das Wickeln des Vorfußes und der Ferse entfallen kann und damit der Patient vorteilhaft in die Lage versetzt wird, den Kompressionsverband eigenhändig und damit auch mit individuellem Kompressionsgefühl, zumindest jedoch ohne ärztliche oder fachlich qualifizierte Hilfe, anzulegen, und darüber hinaus auch bei unterschiedlichen Schwellungszuständen der Extremität nur ein Produkt sowohl für die Akutphase, als auch für die Erhaltungsphase zu benötigen.

Die Lösung der Aufgabe gelingt bei einem Kompressionsverband der im Oberbegriff von Anspruch 1 genannten Art mit der Erfindung dadurch, daß er mit einem fertigen Fußteil ausgebildet ist, welches unterhalb des Knöchels endet.

Der erfindungsgemäße Kompressionsverband mit fertigem Fußteil stellt eine vorteilhafte, weitgehend problemlos anpaßbare Kombination zwischen Kompressionsstrumpf und Kompressionsbinde dar. Damit wird beim Anlegen die Schwierigkeit überwunden, die beim Abwickeln des Vorfußes und der Ferse bisher vorlag, weil diese Bereiche die am schwierigsten zu wickelnden Körperregionen darstellen.
Weil nunmehr der Patient ohne ärztliche oder fachlich qualifizierte Hilfe nach Anziehen des Strumpf teiles in der Art eines gewöhnlichen Strumpfes in der Lage ist, den weiteren Unterschenkelbereich zwischen der Knöchelregion und dem Knie eigenhändig und mit individuell erfühlbarer Kompressionswirkung zu wickeln, wird mit Vorteil eine einfache Handhabung des Kompressionsverbandes mit Anlegbarkeit auch bei variierenden Wadenumfängen und ohne fremde Hilfe verwirklicht.
Somit kann darüber hinaus auf das Anlegen eines Kompressionsstrumpfes verzichtet und statt dessen der Kompressionsverband nach der Erfindung bei allen Schwellungszuständen und über den gesamten Behandlungszeitraum eingesetzt werden.

Das Fußteil weist ein rundgestricktes Kompressionsmittelteil mit einer offenen Fußspitze ohne Zeheneinschluß an einem Ende und einer eingestrickten Ferse am anderen Ende auf, und eine Kompressionsbinde, vorzugsweise mit 3 bis 7 m Länge, die sich am beinseitigen Abschluß des Fußteils anschließt.
Dabei ist vorgesehen, daß das Fußteil im Bereich des Knöchels, und zwar unterhalb desselben endet.
Der Vorteil des Kompressionsverbandes nach der Erfindung ergibt sich daraus, daß sie eine Kombination von Kompressionsstrumpf und Kompressionsbinde darstellt.

Das Anlegen des Kompressionsverbandes nach der Erfindung erfolgt durch:
- Anziehen des Fußteils nach Art eines Strumpfes an den Fuß,
- Umwickeln der am Fußteil angeordneten Binde ohne nochmaliges Umschlingen der Ferse, beginnend am Knöchelbereich, mit bevorzugt Halbdeckung der Bindentouren, spiralförmig oder in Achtertouren, und endend etwa zwei Fingerbreit unter der Kniekehle mit einer zirkulären Tour
oder Anlegen des Kompressionsverbandes nach der Erfindung dadurch, daß der verband nach Anziehen des Fußteiles mit doppelter Umschlingung um die Ferse herumgeführt und bevorzugt mit 1/3-Deckung bis unterhalb der Kniekehle mit einer zirkulären Tour endend mit individuell bestimmbarer Kompressionsspannung um den Unterschenkel herumgewickelt wird.

Weitere Einzelheiten, Merkmale und Vorteile der Erfindung ergeben sich aus der nachstehenden Erläuterung einiger in den Zeichnungen schematisch dargestellter Ausführungsbeispiele.

Es zeigen:
FIG.1b bis 1f in perspektivischer Ansicht ein menschliches Bein von Fuß bis Knie mit einer Folge von Arbeitsphasen beim Anlegen des erfindungsgemäßen Kompressionsverbandes ohne nochmaliges Wickeln der Ferse;
FIG.2b bis 2e in perspektivischer Ansicht ein Bein mit einander folgenden Arbeitsphasen beim Anlegen des Kompressionsverbandes mit Wickeln der Ferse.

Der Fußteil des erfindungsgemäßen Kompressionsverbandes hat eine rundgestricktes Kompressionsmittelteil 2 mit an einem Ende einer offenen Fußspitze 3 ohne Zeheneinschluß, und am anderen Ende einer eingestrickten Ferse 4.

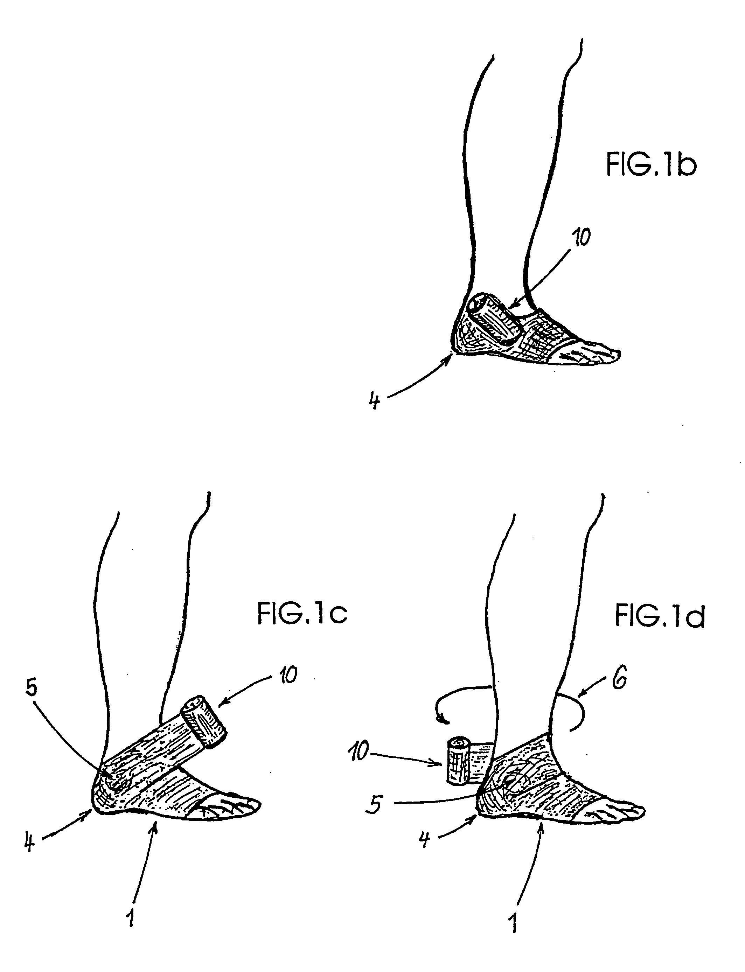

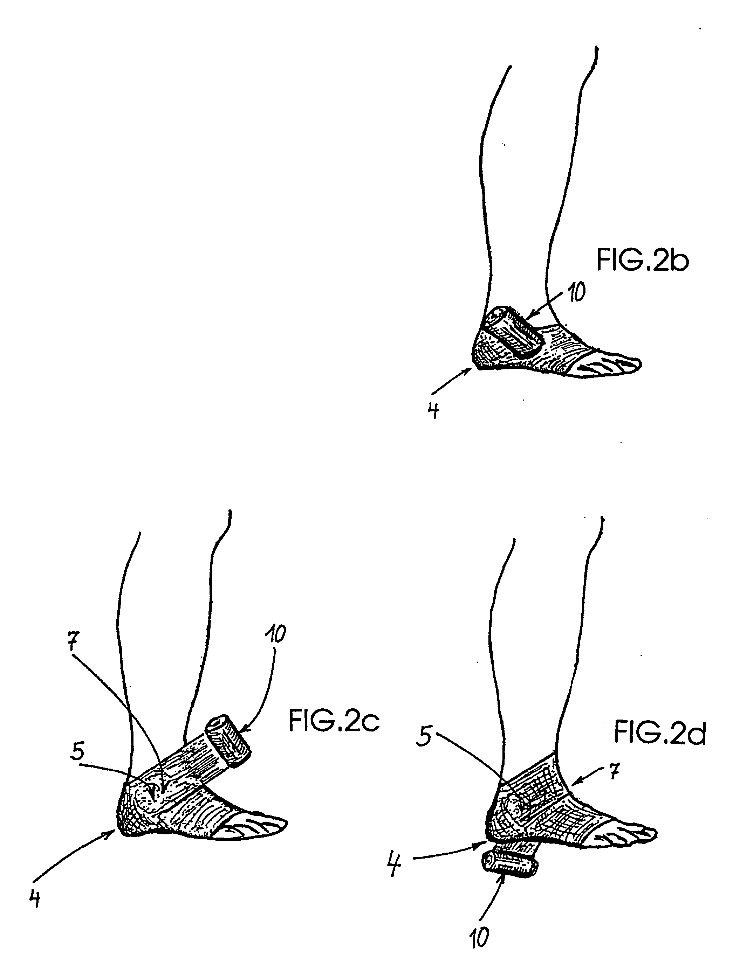

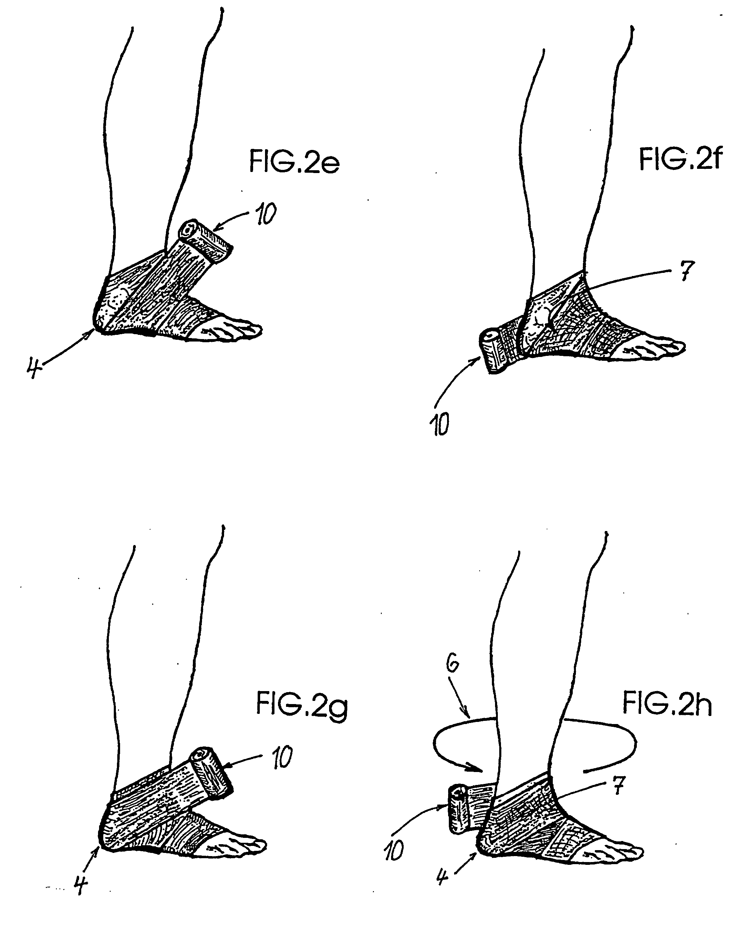

Dazu zeigen die weiteren Figuren 1b bis 1f, daß sich am beinseitigen Abschluß des Fußteils 1 eine Kompressionsbinde 10 mit 3 bis 7 m Länge anschließt.
Das Fußteil 1 endet im Bereich 5 des Knöchels, und zwar unterhalb desselben.
Aus den Darstellungen der Figuren 1b bis 1f, sowie ebenfalls aus den Figuren 2b bis 2e geht deutlich hervor, daß der Kompressionsverband nach der Erfindung eine Kombination von Kompressionsstrumpf und Kompressionsbinde ist.

Das Anlegen des Kompressionsverbandes nach der Erfindung ist außerordentlich einfach. Zunächst wird dabei das Fußteil 1 nach Art eines Strumpfes an den Fuß angezogene. Sodann wird nach den Figuren 1b bis 1f die am Fußteil 1 angeordnete Binde 10 ohne nochmaliges Umschlingen der Ferse 4, beginnend am Knöchelbereich 5, mit bevorzugt ½-Deckung der Bindentouren, spiralförmig oder in Achtertouren, und endend etwa zwei Fingerbreit unter der Kniekehle gemäß dem in FIG.lf gezeigten Endzustand gewickelt.

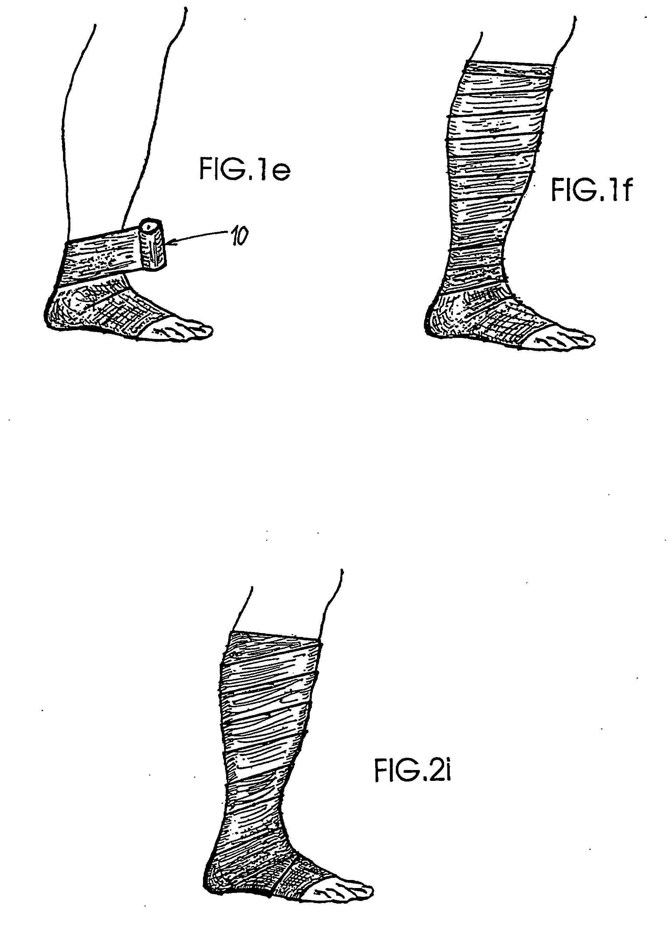

Die Figuren 2b bis 2e zeigen einen ähnlichen Arbeitsvorgang beim Anlegen des erfindungsgemäßen Kompressionsverbandes, der sich lediglich dadurch unterscheidet, daß die Binde 10 nach Anziehen des Fußteils 1 mit doppelter Umschlingung um die Ferse 4 herumgewickelt wird, wie dies den Figuren 2b bis 2g zu entnehmen ist. Dabei wird auch das Fußgelenk 7 und der Bereich 5 des Knöchels, beispielsweise im Falle einer entzündungsbedingten Schwächung des Fußgelenkes 17 erforderlich ist, mit umwickelt und stabilisiert.
Im übrigen ist die Folge der Arbeitsschritte beim Anlegen des erfindungsgemäßen Kompressionsverbandes den Figuren ohne weitere Erläuterungen zu entnehmen. Das fertig umwickelte Bein ist jeweils in den Figuren 1f und 2e gezeigt.

Die Erfindung ist einerseits wegen der einfachen Handhabung und andererseits wegen der vielseitigen Anwendbarkeit eines einzigen Produktes anstelle eines bisher verwendeten Kompressionsstrumpfes bzw. einer Kompressionsbinde infolge ihrer Anlegbarkeit auch bei variierenden Wadenumfängen von großem Vorteil und führt darüber hinaus zu beachtlichen Kostenentlastungen sowohl bei Kostenträgern als auch beim Patienten.

Insofern löst die Erfindung in optimaler Weise die eingangs gestellte Aufgabe.

## Patentansprüche

1. Kompressionsverband zum Stützen des Gewebes im Bereich zwischen Fuß und Knie, beispielsweise zur Behandlung venöser und/oder lymphatischer Schwellungen, mit einem fertigen Fußteil, das ein rundgestricktes Kompressionsmittelteil (2) aufweist und sich am beinseitigen Abschluß des Fußteils (1) eine Kompressionsbinde (10) anschließt **dadurch gekennzeichnet, daß** das Fußteil (1) unterhalb des Knöchels endet.

2. Kompressionsverband nach Anspruch 1, **dadurch gekennzeichnet, daß** das Fußteil (1) eine offene Fußspitze (3) ohne Zeheneinschluß und am anderen Ende eine eingestrickte Ferse (4) aufweist.

3. Kompressionsverband nach Anspruch 1 oder 2, **dadurch gekennzeichnet, daß** die Kompressionsbinde eine Länge von 3 bis 7 m aufweist.

## Claims

1. A compression bandage for supporting the tissue in the area between foot and knee, for example for treating venous and/or lymphatic swellings, with a ready-made foot part which has a circular-knitted compression middle part (2) and with a compression dressing (10) adjoining the leg-side edge of the foot part (1), **characterized in that** the foot part (1) ends below the ankle.

2. The compression bandage as claimed in claim 1, **characterized in that** the foot part (1) has an open front (3) without toe inclusion and, at the other end, a knitted-in heel (4).

3. The compression bandage as claimed in claim 1 or 2, **characterized in that** said compression bandage has a length of 3 to 7 m.

## Revendications

1. Bandage compressif pour soutenir le tissus dans la partie entre le pied et le genou, par exemple pour le traitement d'enflures veineuses et/ou lymphatiques, comprenant une partie finie en pied, qui présente une partie centrale de compression (2) tricotée de manière circulaire et à laquelle se raccorde une bande de compression (10) sur l'extrémité du côté jambe de la partie en pied (1), **caractérisé en ce que** la partie en pied (1) se termine en dessous de la cheville.

2. Bandage compressif selon la revendication 1, **caractérisé en ce que** la partie en pied (1) présente une pointe de pied (3) ouverte n'incluant pas les doigts de pied et un talon (4) tricoté sur l'autre extrémité.

3. Bandage compressif selon la revendication 1 ou 2, **caractérisé en ce que** la bande de compression présente une longueur de 3 à 7 m.
